# EUROPÄISCHE PATENTANMELDUNG

(11) **EP 2 505 159 A1**
(43) Veröffentlichungstag der Anmeldung: **03.10.2012**
(21) Anmeldenummer: 11010192.0
(22) Anmeldetag: 23.12.2011
(51) Int. Cl.: A61B 19/00

(54) **Einrichtung zur Erzeugung von Drehbewegungen, Verfahren, Vorrichtung sowie medizinische Leuchte**

(30) Priorität: 01.04.2011 DE 102011015868; 15.07.2011 DE 102011107779; 15.07.2011 US 201161508087 P
(71) Anmelder: Aevum Mechatronik GmbH, 85478 Garching (DE)
(72) Erfinder: Bachmayer, Mathias, 85748 Garching (DE)
(74) Vertreter: Bobbert & Partner

(57) **Zusammenfassung**

Die vorliegende Erfindung betrifft eine Einrichtung (100) mit wenigstens einem Gelenk (20, 22), welche um das Gelenk (20, 22) drehbar in einem Schwerefeld (G) gelagert ist, wobei die Einrichtung (100) wenigstens einen ersten Abschnitt (10) und wenigstens einen zweiten Abschnitt (12) aufweist, der relativ zum ersten Abschnitt (10) bewegbar ist, dadurch gekennzeichnet, dass der zweite Abschnitt (12) zum Generieren einer Drehbewegung der Einrichtung (100) um das Gelenk (20, 22) ausgestaltet ist. Sie betrifft ferner ein Verfahren zum Bewegen der Einrichtung um das Gelenk, eine Steuereinrichtung, eine Vorrichtung und eine medizinische Leuchte. Des Weiteren betrifft die vorliegende Erfindung ein digitales Speichermedium, ein Computerprogramm-Produkt sowie ein Computerprogramm.

## Beschreibung

Die vorliegende Erfindung betrifft eine Einrichtung mit wenigstens einem Gelenk zum Generieren einer Drehbewegung nach Anspruch 1. Sie betrifft ferner ein Verfahren zum Bewegen der Einrichtung um das Gelenk nach Anspruch 7, eine Steuereinrichtung nach Anspruch 10, eine Vorrichtung nach Anspruch 11 und eine medizinische Leuchte nach Anspruch 12. Des Weiteren betrifft die vorliegende Erfindung ein digitales Speichermedium gemäß Anspruch 13, ein Computerprogramm-Produkt gemäß Anspruch 14 sowie ein Computerprogramm gemäß Anspruch 15.

Kinematische Ketten von Robotern sind aus der Praxis bekannt. Diese weisen regelmäßig zwischen den einzelnen Segmenten des Roboters wirkende Hebel, Seilzüge, Federn, Zahnradgetriebe oder auch Direktantriebe auf.

Eine Aufgabe der vorliegenden Erfindung ist es, eine Einrichtung vorzuschlagen, mit der weitere kinematische Ketten dieser oder anderer Art erzeugt werden können. Ferner sollen ein Verfahren zum Bewegen der Einrichtung um ein Gelenk, eine Steuereinrichtung, eine Vorrichtung, eine medizinische Leuchte, ein geeignetes digitales Speichermedium, ein geeignetes Computerprogramm-Produkt und ein geeignetes Computerprogramm angegeben werden.

Die erfindungsgemäße Aufgabe wird durch eine Einrichtung mit den Merkmalen des Anspruchs 1 gelöst. Sie wird ferner gelöst mittels des Verfahrens zum Bewegen der Einrichtung um das Gelenk nach Anspruch 7, der Steuereinrichtung mit den Merkmalen des Anspruchs 10, der Vorrichtung mit den Merkmalen des Anspruchs 11, der medizinischen Leuchte mit den Merkmalen des Anspruchs 12, des digitalen Speichermediums mit den Merkmalen des Anspruchs 13, des Computerprogramm-Produkts mit den Merkmalen des Anspruchs 14 sowie des Computerprogramms mit den Merkmalen des Anspruchs 15.

Die erfindungsgemäße Einrichtung, im Folgenden auch als Segment bezeichnet, weist einen ersten und einen zweiten Abschnitt auf, wobei der zweite Abschnitt relativ zum ersten Abschnitt bewegbar ist. Der zweite Abschnitt ist zum Erzeugen einer Drehbewegung des Segments um ein Gelenk geeignet und/oder vorgesehen und/oder ausgelegt. Das Segment ist um das Gelenk in einem Schwerefeld drehbar gelagert.

Das erfindungsgemäße Verfahren ist zum Bewegen des erfindungsgemäßen Segments geeignet und/oder vorgesehen. Das Verfahren umfasst den Schritt des Veränderns der Lage des zweiten Abschnitts relativ zum ersten Abschnitt zum Erzeugen oder Generieren einer Drehbewegung des Segments um das Gelenk.

Die erfindungsgemäße Steuereinrichtung, welche eine Regeleinrichtung sein kann, ist geeignet und vorgesehen und/oder konfiguriert zum Ausführen des Verfahrens zum Bewegen des erfindungsgemäßen Segments.

Die erfindungsgemäße Vorrichtung umfasst wenigstens ein erfindungsgemäßes Segment. Die Vorrichtung ist konfiguriert zum Ansteuern von nicht aktuierten Gelenken in dem Segment. Die erfindungsgemäße medizinische Leuchte ist zur Anwendung in einem Operationsraum vorgesehen. Die medizinische Leuchte umfasst wenigstens ein erfindungsgemäßes Segment und/oder eine erfindungsgemäße Steuereinrichtung.

Ein erfindungsgemäßes digitales Speichermedium, insbesondere in Form einer Diskette, CD oder DVD, mit elektrisch auslesbaren Steuersignalen kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein erfindungsgemäßes Computerprogramm weist einen Programmcode auf zur Veranlassung der maschinellen Schritte eines erfindungsgemäßen Verfahrens, wenn das Computerprogramm auf einem Computer abläuft.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll bestimmte erfindungsgemäße Ausführungsformen erläutern.

Ein Schwerefeld ist in einigen erfindungsgemäßen Ausführungsformen das Gravitationsfeld der Erde, auch als Erdanziehungskraft bezeichnet. Dieses Schwerefeld übt somit eine Kraft auf das Segment bzw. alle Elemente oder Komponenten des Segments aus.

In manchen erfindungsgemäßen Ausführungsformen ist das Gelenk ein rotatorisches oder sphärisches Gelenk. In bestimmten erfindungsgemäßen Ausführungsformen weisen rotatorische oder sphärische Gelenke Achsen auf, die als Rotationsachsen oder Drehachsen bezeichnet werden.

In bestimmten erfindungsgemäßen Ausführungsformen werden, insbesondere kontrollierbare, Drehbewegungen des Segments um wenigstens ein Gelenk des Segments durch bewegbare Abschnitte, die das Segment aufweist, erzeugt.

In bestimmten erfindungsgemäßem Ausführungsformen werden die bewegbaren Abschnitte des Segments als zweite Abschnitte bezeichnet. Die zweiten Abschnitte sind relativ zu dem oder den ersten Abschnitten des Segments bewegbar.

In manchen erfindungsgemäßen Ausführungsformen werden die Drehbewegungen des Segments ausschließlich durch die Bewegung der bewegbaren Abschnitte erzeugt, ohne dass die Gelenke, direkt oder, ggf. im Übrigen, indirekt, aktuiert werden.

In einigen erfindungsgemäßen Ausführungsformen sind wenigstens ein Gelenk oder alle Gelenke der Einrichtung nicht aktuiert.

Der Begriff "aktuiert" bedeutet in bestimmten Ausführungsformen "aktiv gesteuert und/oder geregelt". Beispielsweise kann ein aktuiertes Gelenk durch einen Motor oder durch einen mechanischen und/oder elektrischen Antrieb aktiv bewegt werden.

Das Gelenk ist in manchen erfindungsgemäßen Ausführungsformen mit einem steuer- und/oder regelbaren Antrieb verbunden bzw. gekoppelt.

Ein nicht aktuiertes Gelenk kann passive Bewegungen ausführen. Das Gelenk oder wenigstens eine hieran beteiligte Struktur wird dann beispielsweise aufgrund von Kräften und Momenten, die nicht direkt auf das Gelenk wirken, bewegt.

In manchen erfindungsgemäßen Ausführungsformen weist das Segment räumlich bewegbare Abschnitte, im Folgenden auch als Körper bezeichnet, auf. Ein Segment kann einen Körper, oder zwei oder mehr Körper aufweisen.

Der Begriff "Bewegen" bedeutet in bestimmten Ausführungsformen ein Verschieben im Sinne von translatorischem Verschieben und/oder ein Drehen im Sinne von rotatorischem Drehen.

Räumlich bewegbare Körper können im Raum verschiebbare und/oder drehbare Körper sein. Sie können aktuierbar sein.

Aktuierbare Körper können Kräfte und/oder Momente erfahren oder übertragen bekommen, beispielsweise ausgeübt von Motoren (z. B. von Schrittmotoren oder Linearmotoren).

In manchen erfindungsgemäßen Ausführungsformen werden die Drehbewegungen des Segments durch die bewegbaren Körper oder deren Bewegung generiert oder ursächlich erzeugt oder hervorgerufen. In bestimmten erfindungsgemäßen Ausführungsformen werden die Drehbewegungen des Segments ausschließlich durch die Bewegung der bewegbaren Körper generiert oder ursächlich erzeugt oder hervorgerufen.

Drehbewegungen des Segments um die Drehachse oder um mehrere Drehachsen des Gelenks des Segments können durch translatorische oder rotatorische Körper hervorgerufen werden, die sich in oder an dem Segment bewegen.

In bestimmten erfindungsgemäßen Ausführungsformen weist das Segment Mittel, z. B. Körper, zur Erzeugung von - insbesondere kontrollierbaren oder kontrollierten - Beschleunigungen des Segments auf. Das Segment kann dabei in oder um eine(r) oder mehrere(n) Drehachsen des Gelenks gedreht werden.

In bestimmten erfindungsgemäßen Ausführungsformen weist das Segment Mittel zum aktiven Verändern, Steuern oder Regeln der Lage des bewegbaren zweiten Abschnitts relativ zum ersten Abschnitt auf. Derartige Mittel können beispielsweise mechanische oder auf bestimmten physikalischen Effekten beruhende (z. B. Magnetismus bzw. magnetische) Antriebsmechanismen sein, die den zweiten Abschnitt bewegen. In einigen erfindungsgemäßen Ausführungsformen erfolgt der Antrieb mittels eines ferngesteuerten Motors, der auf dem zweiten Abschnitt angeordnet ist.

In bestimmten erfindungsgemäßen Ausführungsformen ist der bewegbare zweite Abschnitt relativ zum ersten Abschnitt verschiebbar und/oder rotierbar mit der Einrichtung verbunden.

In bestimmten erfindungsgemäßen Ausführungsformen weist das Segment wenigstens zwei aktuiert miteinander verbundene Körper auf. Die Körper können mechanisch - beispielsweise mittels Riemen oder Zahnrad - miteinander verbunden sein.

In bestimmten erfindungsgemäßen Ausführungsformen weist das Segment wenigstens ein aktuiertes Gelenk auf.

Eine Drehbewegung des Segments kann durch das aktuierte Gelenk vollständig oder teilweise ausgeführt oder unterstützt werden. Beispielsweise kann die Drehung durch das aktuierte Gelenk und/oder einen translatorischen Körper und/oder einen rotatorischen Körper des Segments ausgeführt werden.

Das erfindungsgemäße Verfahren zum Bewegen des Segments umfasst in gewissen erfindungsgemäßen Ausführungsformen das Verändern der Lage des bewegbaren Körpers (zweiter Abschnitt) relativ zum ersten Abschnitt zum Generieren einer Drehbewegung der Einrichtung um das Gelenk.

Der Begriff "Bewegen" wird in manchen erfindungsgemäßen Ausführungsformen synonym zu dem Begriff "Manipulieren" verwendet.

Der Begriff "Manipulieren" ist in bestimmten Ausführungsformen als Ansteuern und/oder Verändern zu verstehen. Ein manipuliertes Gelenk kann z. B. gedreht, verschoben, blockiert, in gezielter Weise geregelt oder gesteuert, aktuiert oder in einer anderen Art und Weise beeinflusst werden.

Das erfindungsgemäße Verfahren zum Bewegen von wenigstens einem nicht aktuierten Gelenk in dem Segment umfasst in bestimmten Ausführungsformen das Bestimmen der Winkel und/oder der Positionen der aktuierten und/oder der nicht aktuierten Gelenke des Segments; das Vergleichen dieser Winkel und/oder Positionen mit vorgegebenen Sollwerten; das Bestimmen einer Abweichung zwischen den gemessenen Werten und Sollwerten und ein anschließendes Verändern der Positionen und/oder Geschwindigkeiten der aktuierten Gelenke aufgrund einer Abweichung.

Der Begriff "Bestimmen" bedeutet in bestimmten Ausführungsformen ein direktes oder indirektes, z. B. mittels Parametern, Messen von physikalischen Größen. "Bestimmen" kann auch ein mathematisches Bestimmen aufgrund von funktionalen Beziehungen physikalischer Größen bedeuten. Ferner können "zu bestimmende Werte" aus Tabellen oder abgespeicherten Daten ermittelt werden.

Die Winkel und/oder Positionen der Gelenke, sowohl der aktuierten als auch der nicht aktuierten Gelenke, können z. B. durch Messverfahren (Winkelmessverfahren, Koordinaten-Positionsmessungen, etc.) bestimmt werden. Sie können jedoch auch anhand bestimmter Parameter durch Tabellen, Kalibrierkurven etc. bestimmt werden. Insbesondere können zu Beginn jeder Bewegung, sowohl der Körper als auch des Segments, die inertialen Winkel oder Ausgangswinkel der Gelenke bestimmt werden.

Bei aktuierten Gelenken können die Winkel und/oder Positionen in einigen erfindungsgemäßen Ausführungsformen auch direkt aus dem Aktuierungsmechanismus bestimmt werden, sofern dieser Mechanismus dazu geeignet ist (z. B. Drehgeber-Messverfahren, angeschlossene Winkelmessgeräte, etc.).

Anschließend können die zuvor bestimmten Winkel und/oder Positionen mit vorgegebenen Sollwerten verglichen werden. Sollwerte können beispielsweise Wegkurven, Drehwinkel, etc. sein, die das Segment nach vorgegebenen Werten (aus Tabellen, gespeicherten Sollwerten etc.) erreichen soll.

Weichen die zuvor bestimmten Winkel und/oder Positionen von den Sollwerten ab, so können die Position und/oder die Geschwindigkeit der aktuierten und/oder der nicht aktuierten Gelenke aufgrund einer Abweichung verändert werden, wie dies in einigen erfindungsgemäßen Ausführungsformen vorgesehen ist. Diese Veränderung kann mittels Zustandsregelungs-Verfahren durchgeführt werden.

Das erfindungsgemäße Verfahren umfasst in bestimmten Ausführungsformen das Bestimmen von wenigstens einer Kraft, z. B. einer Lagerkraft, die auf wenigstens eines der aktuierten oder nicht aktuierten Gelenke des Segments wirkt; das Vergleichen dieser Kraft mit vorgegebenen Sollwerten; Bestimmen einer Abweichung zwischen den zuvor bestimmten (z. B. gemessenen) Werten und den Sollwerten; und das mathematische Bestimmen von Sollwinkeltrajektorien aufgrund der bestimmten Abweichung; Festlegen und Ausführen einer Bewegung von an dem zweiten Abschnitt angreifenden Aktoren der Einrichtung entsprechend den Sollwinkeltrajektorien.

Der Begriff "Aktor" ist in bestimmten Ausführungsformen ein Motor, Stellmotor, Schrittmotor, allgemein ein mechanischer Antrieb oder ähnliches.

In manchen erfindungsgemäßen Ausführungsformen wirkt der Aktor auf ein aktuiertes Element (z. B. auf ein Gelenk) mittels einer Übersetzung wie beispielsweise einem Zahnrad, einem Getriebe, einem Riemen (z. B. ein Zahnriemen), usw.

Das Manipulieren der Bewegung und/oder der Beschleunigung der nicht aktuierten Gelenke des Segments ist in einigen erfindungsgemäßen Ausführungsformen eine gesteuerte und/oder geregelte Beeinflussung der nicht aktuierten Gelenke, insbesondere durch kontrollierte Bewegungen der Körper im oder auf dem Segment.

Die Kräfte der nicht aktuierten Gelenke, insbesondere die Lagerkräfte der Gelenke, können durch Messen der Richtung und des Betrags der Kräfte mittels dem Fachmann bekannter Verfahren bestimmt werden.

In manchen erfindungsgemäßen Ausführungsformen führen die Segmente räumliche Bahnkurven aus, die als Sollwinkeltrajektorien vorgegeben sind. Diesen räumlichen Bahnkurven können die Aktoren auf dem Segment (z. B. Körper mit Aktoren oder aktuierte Körper) folgen, um die nicht aktuierten Gelenke gezielt zu steuern. Anders ausgedrückt erfolgt eine indirekte Steuerung bzw. Bewegung der nicht aktuierten Gelenke durch Aktoren auf dem Segment.

Das erfindungsgemäße Verfahren umfasst in bestimmten Ausführungsformen das Bestimmen der Winkel und/oder Positionen wenigstens eines der aktuierten oder nicht aktuierten Gelenke des Segments; das Bestimmen der Positionen und/oder Geschwindigkeiten der Körper in dem Segment; das Bestimmen der Positionen und/oder Geschwindigkeiten der Körper in dem Segment zueinander (relative Positionen und/oder Geschwindigkeiten der Körper in den Segmenten zueinander); und das Verändern der Positionen und/oder Geschwindigkeiten der Körper des Segments zueinander aufgrund der gemessenen Winkel und/oder Positionen der aktuierten Gelenke des Segments.

In bestimmten erfindungsgemäßen Ausführungsformen umfasst das erfindungsgemäße Verfahren das Bestimmen der Winkel und/oder Geschwindigkeiten der nicht aktuierten Gelenke des Segments; das Erzeugen von Steuersignalen für die aktuierten Körper des Segments aufgrund der gemessenen Winkel und/oder Geschwindigkeiten; und das Ansteuern der aktuierten Körper des Segments mittels der erzeugten Steuersignale. Steuersignale können in einigen erfindungsgemäßen Ausführungsformen elektrische Spannungen und/oder elektrische Ströme sein. Diese können Motoren in geeigneter Weise steuern, beispielsweise um bestimmte Drehungen zu erzeugen.

Steuersignale können Vorsteuersignale sein.

Das "Ansteuern" der aktuierten Körper kann das Bewegen der aktuierten Körper mittels der Steuersignale bedeuten.

In bestimmten erfindungsgemäßen Ausführungsformen umfasst das erfindungsgemäße Verfahren das Bestimmen der Winkel ünd/oder Winkelgeschwindigkeiten der nicht aktuierten Gelenke des Segments; das Bestimmen einer Differenz zwischen einem vorgegebenen Sollwert einer Aktorstellgröße und einer aktuellen Aktorstellgröße; optional das Verstärken dieser Differenz in proportionaler und/oder integraler und/oder differentieller Form; das Erzeugen von Steuersignalen für die aktuierten Körper des Segments aufgrund der bestimmten Winkel und/oder Geschwindigkeiten und/oder bestimmten Differenzen; und das Ansteuern der aktuierten Körper des Segments mittels der erzeugten Steuersignale.

In manchen erfindungsgemäßen Ausführungsformen tritt eine Differenz zwischen aktuellen Messwerten von bestimmten, z. B. gemessenen, Winkeln und/oder Geschwindigkeiten und vorgegebenen Sollwerten nicht aktuierter Gelenke auf. Die Ursache kann beispielsweise ein zeitverzögertes Messen physikalischer Parameter sein oder eine zeitverzögerte Weiterleitung von vorgegebenen Sollwerten aus einem Datenspeicher. Das Verfahren zum Manipulieren von nicht aktuierten Gelenken kann jedoch eine hohe Dynamik erfordern, die durch zeitverzögerte Prozesse erschwert wird. Erfindungsgemäß kann diese Differenz durch einen sogenannten PID-Verstärker zu einem Aktor eines aktuierten Gelenks rückgeführt werden. Damit können Steuersignale oder Vorsteuersignale entsprechend einer vorgegebenen Dynamik erzeugt und zum Ansteuern beispielsweise von aktuierten Körpern des Segments weitergeleitet werden.

In bestimmten erfindungsgemäßen Ausführungsformen umfasst das erfindungsgemäße Verfahren das Bestimmen der Drehraten einzelner und/oder aller Körper der Segmente; das Erzeugen von Steuersignalen für die aktuierten Körper des Segments aufgrund der bestimmten Winkel und/oder Geschwindigkeiten; und das Ansteuern der aktuierten Körper des Segments mittels der erzeugten Steuersignale.

In bestimmten erfindungsgemäßen Ausführungsformen umfasst das erfindungsgemäße Verfahren das Bestimmen der Drehraten einzelner und/oder aller Körper der Segmente; das Bestimmen einer Differenz zwischen einem vorgegebenen Sollwert einer Aktorstellgröße und der aktuellen Aktorstellgröße; das Verstärken dieser Differenz in proportionaler und/oder integraler und/oder differentieller Form, beispielsweise mittels eines PID-Verstärkers; das Erzeugen von Steuersignalen für die aktuierten Körper des Segments aufgrund der bestimmten Winkel und/oder Geschwindigkeiten und/oder bestimmten Differenzen; und das Ansteuern der aktuierten Körper des Segments mittels der oder mit den erzeugten Steuersignalen.

In bestimmten erfindungsgemäßen Ausführungsformen umfasst das erfindungsgemäße Verfahren zum Bestimmen von Solltrajektorien für aktuierte Gelenke in einem Segment das Bestimmen von Sollwinkeln nicht aktuierter Gelenke des Segments; das Bestimmen von Sollwinkelgeschwindigkeiten aus den zuvor bestimmten Sollwinkeln nicht aktuierter Gelenke des Segments; das Erzeugen von Regelungssignalen und/oder Steuersignalen für die aktuierten Gelenke des Segments aufgrund der bestimmten Sollwinkel und/oder Sollwinkelgeschwindigkeiten; und das Ansteuern der aktuierten Körper des Segments mit den erzeugten Regelungssignalen und/oder Steuersignalen.

In manchen erfindungsgemäßen Ausführungsformen ist das Verfahren zum Bestimmen (z. B. durch Berechnen) von Solltrajektorien für die aktuierten Gelenke in einem Segment ein Regelungs- und/oder Steuerverfahren.

Die erfindungsgemäße Vorrichtung, welche hierin auch als kinematische Kette bezeichnet wird, ist in bestimmten erfindungsgemäßen Ausführungsformen ein Roboterarm.

In manchen erfindungsgemäßen Ausführungsformen ist die kinematische Kette eine geregelte und/oder gesteuerte serielle oder parallele Roboterarmkinematik.

In bestimmten erfindungsgemäßen Ausführungsformen ist mindestens ein Aktor der kinematischen Kette gegen Eintritt von Flüssigkeit oder hermetisch dicht ausgestaltet.

Eine solche Ausgestaltung, insbesondere eine hermetisch dichte Ausgestaltung, kann gegen äußere Einflüsse wie beispielsweise Feuchtigkeit, Spritzwasser, Wasser allgemein, Fett, Öl, Schmutz, Staub etc. schützen.

In einigen erfindungsgemäßen Ausführungsformen der Einrichtung oder der Vorrichtung sind die Gelenkfreiheitsgrade einiger oder aller der zueinander beweglichen Elemente nicht durch eine mechanisch vorhandene kinematische Bindung vorgegeben. Dies ist beispielspielsweise bei manchen aus der Praxis bekannten Baukränen anders.

Erfindungsgemäße Ausführungsformen weisen einen oder mehrere der oben oder im Folgenden genannten Vorteile auf.

Alle Vorteile, die für die erfindungsgemäßen Einrichtungen und die erfindungsgemäßen Vorrichtungen zutreffen, sind ungeschmälert auch mit dem erfindungsgemäßen Verfahren erzielbar. Um Wiederholungen zu vermeiden wird auf die Beschreibung derselben in den obigen Abschnitten verwiesen.

Die vorliegende Erfindung betrifft eine Einrichtung mit Abschnitten zur Erzeugung eines antreibenden Momentes in Folge einer konfigurierbaren Massenverteilung der Abschnitte in der Einrichtung als dem zu bewegenden Körper. Im Gegensatz zu klassischen Kinematikkonzepten der Robotik mit zu bewegenden Einzelsegmenten ist hier vorteilhaft keine direkte mechanische, kapazitive oder magnetische Kopplung der Segmente zur Momenten- oder Kraftübertragung benachbarter Segmente notwendig.

Neben den klassischen seriellen Roboterkinematiken (seriell bewegte Segmente) gibt es parallele Roboterkinematiken. Dabei wirken Hebel, Seilzüge, Federn, Zahnradgetriebe oder Direktantriebe zwischen den einzelnen Segmenten des Roboters. Aufgrund der Schwerkraft und der in vielen Anwendungsfällen geforderten Dynamik sind aus der Praxis bekannte Antriebe oftmals derart ausgelegt, dass eine Verletzungsgefahr für den Menschen besteht. Möchte man dem entgegenwirken, ist dies mit einer zusätzlichen Sensorik verbunden, was kostenintensiv ist und bzgl. der Zuverlässigkeit immer ein Restrisiko bestehen lässt. Besonders in einem Umfeld, in dem ein Roboter mit einem Menschen interagiert, ist es daher vorteilhaft, eine Einrichtung zu verwenden, die dem Menschen auch bei Versagen der steuernden Elektronik keinen Schaden zufügen kann. Dies wird mit der erfindungsgemäßen Einrichtung vorteilhaft erreicht.

Zum Vermeiden von unkontrollierten Bewegungen dieser Antriebsbauteile oder der angetriebenen Bauteile sind erfindungsgemäß zusätzliche Sensoren nicht erforderlich, was einen weiteren Vorteil darstellen kann.

Besonders bei aktuierten Haushalts- oder Bürohilfen, wie z. B. bei aktuierten Schreibtischlampen, kann die erfindungsgemäße Einrichtung zum Schutz von Personen vorteilhaft eingesetzt werden.

Die erfindungsgemäße Einrichtung kann vorteilhaft bei derartigen Anwendungen eingesetzt werden, bei denen es weniger auf hohe Dynamik und Positioniergüte ankommt, dafür um so mehr darauf, die maximal auftretende Kraft zu begrenzen. Die Aktuierung von Vorrichtungen mit einem derartigen Anforderungsprofil wäre eine mögliche Anwendung der vorliegenden Erfindung.

Ein weiteres vorteilhaftes Anwendungsgebiet der erfindungsgemäßen Einrichtung liegt in Bereichen, in welchen es darauf ankommt, hermetisch dichte Manipulatoren zu verwenden, beispielsweise, um entweder nicht zu tolerierende Verunreinigungen im umgebenden Medium in Folge von z. B. Lagerölen zu vermeiden, oder weil im Arbeitsumfeld aggressive Substanzen vorhanden sind, die Dichtungsmaterialien angreifen und zerstören könnten. Mittels der erfindungsgemäßen Einrichtung können in einem Schwerefeld, wie beispielsweise dem Gravitationsfeld der Erde, arbeitende Manipulatoren hermetisch dicht eingesetzt werden. Hierzu weisen sie in bestimmten erfindungsgemäßen Ausführungsformen eine autonome Energieversorgung (z. B. Batterie/Akku) auf. Zudem sind sie in einigen erfindungsgemäßen Ausführungsformen vorteilhaft gegenüber der Umgebung abgedichtet, beispielsweise sind sie in einer Edelstahlhülle verkapselt (verschweißt).

Die Erfindung kann Segmente umfassen, die aus mehreren aktuiert gelenkig miteinander verbundenen Körpern bestehen, die als eigenes kinematisches System ein Segment in einer kinematischen Kette bilden können, bei der keines der Gelenke, welche die beteiligten Segmente verbinden, aktuiert sein muss. Dies verringert vorteilhaft die Anzahl der Aktoren der kinematischen Kette. Hierdurch können das Gewicht und die Komplexität des Gesamtsystems vorteilhaft verringert werden. Dennoch können die Gelenke der nicht aktuierten, die Segmente jeweils lagernden Gelenke gezielt angesteuert werden, beispielsweise mittels steuerungs- oder regelungstechnischer Verfahren.

Bei Verwendung derartiger Segmente zum Aufbau kinematischer Ketten ist es vorteilhaft möglich, unteraktuierte Roboterarme aufzubauen, die bezüglich des relevanten kinematischen Pfads vom Einspannungsort bis zur Position des Werkzeugs (diese Position des Werkzeugs wird auch als "Tool Center Point", abgekürzt TCP, bezeichnet) die Eigenschaften eines vollaktuierten Arms aufweisen. Die maximal auftretenden Momente können dadurch vorteilhaft reduziert werden. Der Einsatz im Umfeld des Menschen ist vorteilhaft ungefährlich möglich, z. B. bei Anwendung kinematischer Ketten als Haushalts- oder Bürohilfe.

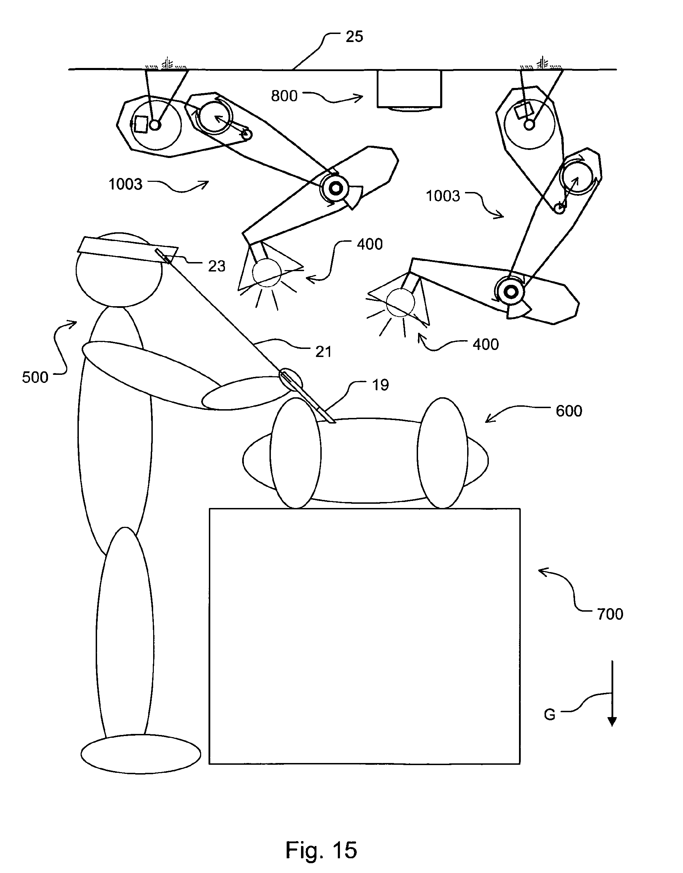

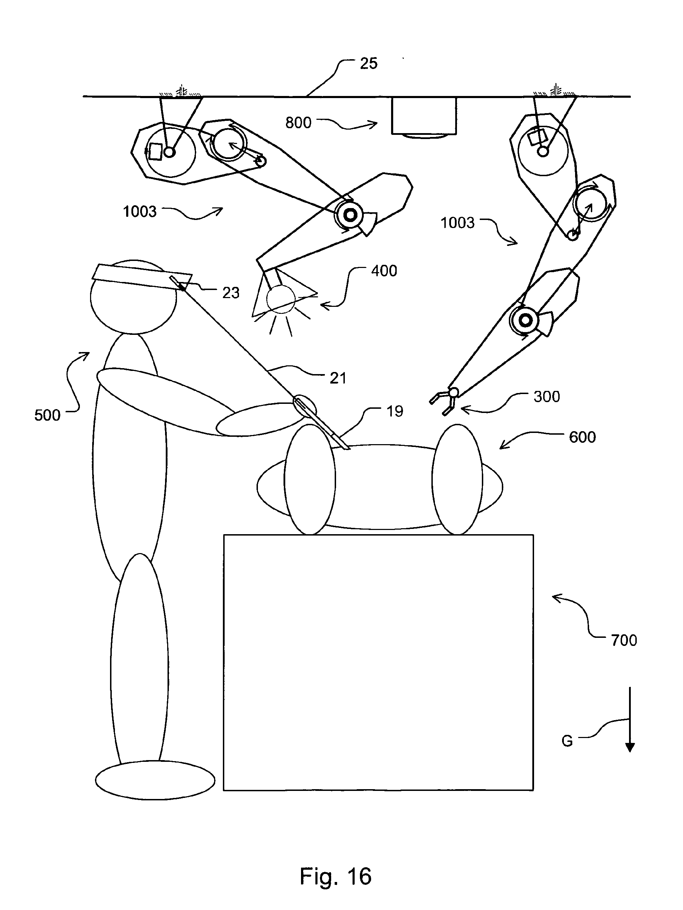

Weitere vorteilhafte Anwendungen sind in der Medizintechnik möglich. Bei den erfindungsgemäßen Ausführungsformen, wie sie in den Figuren 15 und 16 in Operationsräumen zur Behandlung von Patienten gezeigt werden, kann vorteilhaft die Sicherheit für den Chirurgen und/oder den Patienten erhöht werden. Beispielsweise können bei einem Ausfall elektrischer Systeme zur Steuerung oder Regelung der kinematischen Ketten oder bei einem vollständigen Stromausfall die kinematischen Ketten (die auch als Roboterarme bezeichnet werden) vorteilhaft per Hand (des Chirurgen oder anderer Personen) verschoben werden, um beispielsweise bestimmte notwendige Schritte im Operationsgebiet zu beenden. Dieses Verschieben der Roboterarme ist dadurch möglich, dass die Roboterarme nach dem Funktionsprinzip momentenbegrenzt sind. Daher kann ein defekter Roboterarm einfach per Hand zur Seite geschoben werden.

Gegenüber dem Aufbau und der Funktionsweise von Baukränen weist die vorliegende Erfindung mehrere Vorteile auf, die im Folgenden beschrieben werden.

Baukräne verfügen über Gegengewichte, um den Schwerpunkt des Geräts innerhalb der Mastgrundfläche zu halten. Jedoch wird die geometrische Lage des Arms nicht durch eine geregelte und kontrollierte Bewegung des Gegengewichts, sondern durch eine kinematische Bindung, z. B. mittels Seil oder Stange, realisiert. Dies ist in manchen erfindungsgemäßen Ausführungsformen vorteilhaft nicht der Fall.

Ferner dient das Gegengewicht der bekannten Kranlösungen nicht dazu, eine Bewegung des Arms relativ zum Mast zu verursachen, sondern lediglich dazu, den Schwerpunkt des Arms über der durch die Standfußpunkte aufgespannten Fläche des Krans zu halten, um ein Umkippen des gesamten Krans zu verhindern. Dies ist in bestimmten erfindungsgemäßen Ausführungsformen vorteilhaft nicht der Fall und/oder nicht erforderlich.

Nachfolgend werden Ausführungsbeispiele der vorliegenden Erfindung unter Bezugnahme auf die beigefügten Figuren rein exemplarisch beschrieben. Dabei werden die vier mit dieser Erfindung zur Erzeugung einer kontrollierbaren Drehbewegung verbundenen Grundrealisationsprinzipien anhand eines Segments eines Roboters dargestellt. Nachfolgend gegebene Freiheitsgrade der Rotation oder Translation können manuell, oder aber auch aktuiert durch einen entsprechend ausgeführten Antrieb betätigt werden. Dabei existiert in manchen erfindungsgemäßen Ausführungsformen keine direkte Kraft- oder Momentenübertragung bzgl. des freien Lagerfreiheitsgrades zwischen den beiden Körpern. Zur koordinierten Bewegung des Gebildes kann die inertiale Lage der einzelnen Gelenke erfasst und durch eine entsprechende Regelung der zusätzlich vorhandenen Körper die Dynamik des Gesamtkonstrukts in gewünschter Weise beeinflusst werden.

Im Folgenden wird die erfindungsgemäße Vorrichtung anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt eine Einrichtung, aufweisend einen Grundkörper und einen darauf translatorisch gelagerten Abschnitt;
- Fig. 2: zeigt eine Einrichtung, aufweisend einen Grundkörper und einen darauf drehbaren und/oder verschiebbaren Abschnitt;
- Fig. 3: zeigt Fig. 2, zusätzlich eine ausgeprägte Unwucht aufweisend;
- Fig. 4: zeigt eine Einrichtung, aufweisend einen Grundkörper und eine Pumpe mit einem Steuergerät zum Umverteilen von Flüssigkeit zwischen verschiedenen Vorratsvolumina;
- Fig. 5: zeigt eine Einrichtung, aufweisend einen Grundkörper und verschiedene Abschnitte, wie sie in den Figuren 1 bis 3 dargestellt wurden;
- Fig. 6: zeigt eine kinematische Kette, aufweisend drei Einrichtungen entsprechend Fig. 1;
- Fig. 7: zeigt eine weitere kinematische Kette, aufweisend drei Einrichtungen entsprechend Fig. 2;
- Fig. 8: zeigt eine elastisch deformierbare Einrichtung mit einem Grundkörper und einem Abschnitt;
- Fig. 9: zeigt eine weitere kinematische Kette, aufweisend drei Einrichtungen mit einem Manipulator;
- Fig. 10: zeigt eine weitere kinematische Kette, aufweisend drei Einrichtungen mit einer Leuchte;
- Fig. 11: zeigt Fig. 1, jedoch mit variablem Achsabstand zwischen den beiden Gelenken;
- Fig. 12: zeigt Fig. 2, jedoch mit variablem Achsabstand zwischen den beiden Gelenken;
- Fig. 13: zeigt Fig. 3, jedoch mit variablem Achsabstand zwischen den beiden Gelenken;
- Fig. 14: zeigt Fig. 8, jedoch mit variablem Achsabstand zwischen den beiden Gelenken;
- Fig. 15: zeigt eine Anwendung der kinematischen Kette aus Fig. 10 mit einer Operationsleuchte; und
- Fig. 16: zeigt eine weitere Anwendung der kinematischen Kette aus Fig. 10 mit einer Operationsleuchte und mit einem Greifarm.

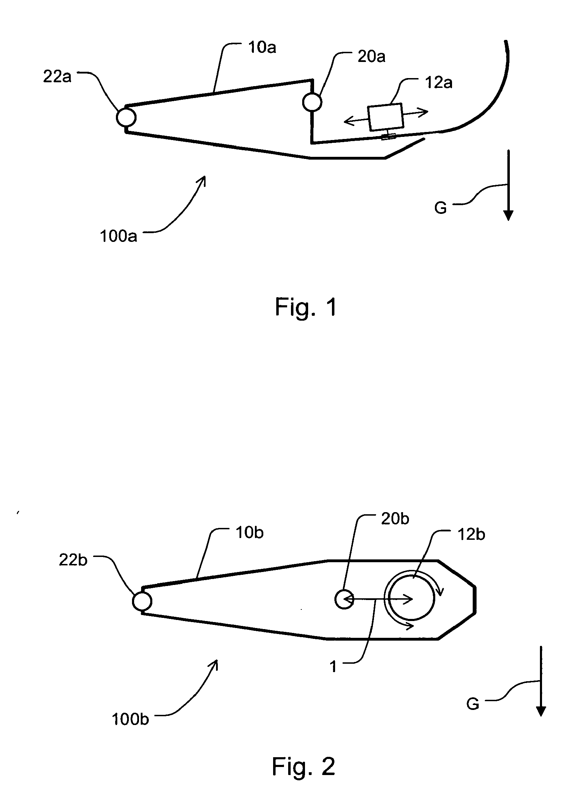

**Fig. 1** zeigt eine Einrichtung 100a (im Folgenden als Segment 100a bezeichnet), mit einem Grundkörper 10a und einem darauf oder daran translatorisch gelagerten Abschnitt 12a (im Folgenden auch als Körper 12a bezeichnet).

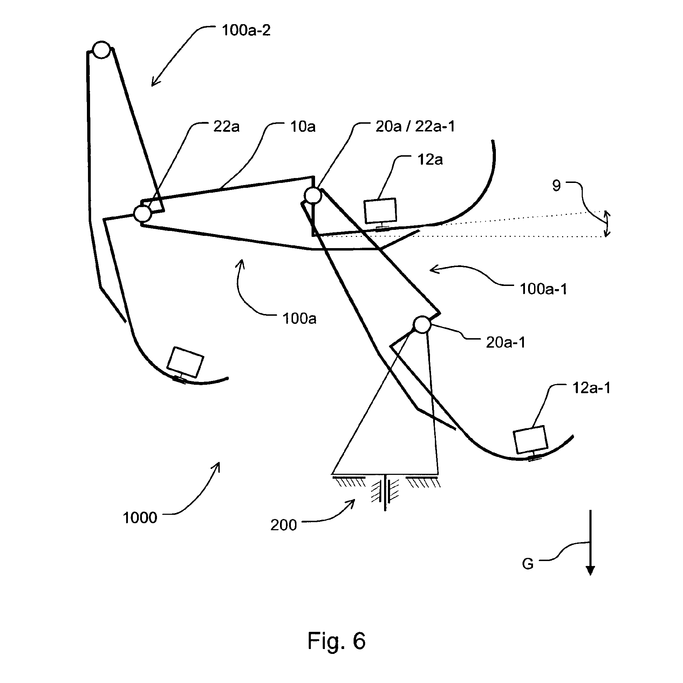

Der Grundkörper 10a weist zwei Gelenke 20a und 22a auf, die auch als Drehgelenkpunkte oder Achsgelenkpunkte bezeichnet werden. Diese Gelenke 20a, 22a ermöglichen es, dass das Segment 100a als Teil bzw. im Zusammenspiel mit anderen Körpern oder Gegenständen in einer kinematischen Kette wirkt (wie in Fig. 6 dargestellt). Eine kinematische Kette kann auch als Roboterarm bezeichnet werden.

Ist der Grundkörper 10a am Ende einer kinematischen Kette angebracht, so reicht das Gelenk 20a für die Funktion in der kinematischen Kette aus. In diesem Fall bedarf es keines weiteren Gelenks 22a.

Gemäß den Regeln der Mechanik stellt sich ein Gesamtschwerpunkt der beteiligten Körper (Grundkörper 10a, Körper 12a) in Abhängigkeit von ihrer räumlichen Lage zueinander ein. Die Gesamtkörper (Grundkörper 10a und Körper 12a) befinden sich rotatorisch in Ruhe, wenn sich ein resultierendes Momentengleichgewicht aus Kräften und Momenten einstellt. Im vorliegenden Fall der Fig. 1 befindet sich der Gesamtkörper dann in Ruhe, wenn sich, bei einem Drehpunkt des Gesamtkörpers um das Gelenk 20a, einerseits das Moment aller angreifenden Kräfte in den Gelenken 20a und 22a um den Drehpunkt 20a und andererseits das Moment des Gesamtschwerpunkts um den Drehpunkt des Gelenks 20a im Gleichgewicht befinden.

Wird der Körper 12a relativ zum Grundkörper 10a verschoben, so stellt sich ein neuer Gesamtschwerpunkt ein. Bei entsprechenden Bewegungen der Körper 10a und 12a im System stellt sich ein neues Gesamtgleichgewicht ein. Die Bahn, entlang welcher sich der Grundkörper 10a bewegt, kann geradlinig, aber auch jede beliebig kurvenförmige Bahn (Kurvenbahn) sein. Durch derartige beliebige Kurvenbahnen kann die Bahn der möglichen Ruhelagen der Gesamtschwerpunkte für den Arbeitsraum optimiert werden.

Der Körper 12a kann mit dem Ziel, den Grundkörper 10a in eine vorbestimmte neue Ruhelage, auch als Sollwert zu verstehen, zu bringen, verschoben werden. Die neue Ruhelage des Grundkörpers 10a wird dabei durch konkrete Werte der Drehwinkel der nicht aktuierten Gelenke 20a und/oder 22a beschrieben. Um die neue Ruhelage zu erreichen, kann eine entsprechend ausgelegte oder konfigurierte Drehratenregelung (Drehrate = Drehgeschwindigkeit oder Drehwinkel pro Zeit) eingesetzt werden. Bei dieser Drehratenregelung werden die Drehwinkel und/oder Drehgeschwindigkeiten der Gelenke 20a und/oder 22a gemessen und mit Sollwerten der Drehwinkel, die der Grundkörper 10a in der neuen Ruhelage einnehmen oder aufweisen soll, verglichen bzw. verrechnet. Daraus werden neue Stellgrößen für den aktuierten Körper 12a des Segments 100a abgeleitet.

Alternativ zur Drehratenregelung kann der aktuierte Körper 12a in gesteuerter Weise basierend auf den jeweils gemessenen Positionen und/oder Geschwindigkeiten des aktuierten Körpers 12a die nicht aktuierten Gelenke (20a, 22a) beeinflussen.

Der aktuierte Körper 12a kann bzgl. seiner Positionen und/oder Bewegungen zusätzlich einen eigenen Positionsregelkreis aufweisen.

**Fig. 2** zeigt eine Einrichtung 100b (auch als Segment 100b bezeichnet), mit einem Grundkörper 10b und einem relativ zum Grundkörper 10b drehbaren und/oder verschiebbaren Abschnitt 12b (Körper 12b). Der Verschiebeweg 1 des Abschnitts 12b entspricht dem Abstand zwischen der Drehachse des Körpers 12b und der Drehachse des Gelenks 20b.

Es wird eine unwuchtfreie Drehung des Grundkörpers 10b um die Drehachse des Gelenks 20b vorausgesetzt. Dabei wird nur dieser eine rotatorische Freiheitsgrad des Grundkörpers 10b angenommen.

Der Grundkörper 10b verfügt über mindestens zwei Gelenke 20b und 22b, wenn er im Zusammenspiel in einer kinematischen Kette wirken soll (wie in Fig. 6 dargestellt).

Ist der Grundkörper 10b am Ende einer kinematischen Kette angebracht, so reicht das Gelenk 20b für die Funktion in der kinematischen Kette aus. Ein weiteres Gelenk 22b ist nicht erforderlich.

Gemäß den Regeln der Mechanik stellt sich wiederum ein Gesamtschwerpunkt der beteiligten Körper (Grundkörper 10b, Körper 12b) in Abhängigkeit von ihrer räumlichen Lage zueinander ein. Die Gesamtkörper (Grundkörper 10b und Körper 12b) befinden sich rotatorisch in Ruhe, wenn sich ein resultierendes Momentengleichgewicht aus Kräften und Momenten einstellt. Im vorliegenden Fall der Fig. 2 befindet sich der Gesamtkörper dann in Ruhe, wenn sich in einem Drehpunkt des Gesamtkörpers um das Gelenk 20b einerseits das Moment aller angreifenden Kräfte in den Gelenken 20b und 22b um den Drehpunkt 20b und andererseits das Moment des Gesamtschwerpunkts um den Drehpunkt des Gelenks 20b im Gleichgewicht befinden.

Wird der Körper 12b rotatorisch bewegt und/oder beschleunigt, wirkt sich das hieraus ergebende Moment störend auf das Momentengleichgewicht aus, und der Grundkörper 10b beginnt sich zu drehen. Ist der Grundkörper 10b zudem translatorisch relativ zum Körper 12b beweglich, so können Lastwechsel, die beispielweise im Gelenk 22b auftreten, kompensiert werden. Ferner kann eine Bewegung des Grundkörpers 10b, z. B. eine Drehung um die Achse des Gelenks 20b, durch eine translatorische Verschiebung des Körpers 12b entsprechend Fig. 1 bewirkt werden.

Liegt die translatorische Bahn (Verschiebeweg 1) des Körpers 12b in der Ebene des Schwerpunkts des Grundkörpers 10b und eines Gelenks (z. B. des Gelenks 20b im Falle serieller Kinematik), so sind durch Zusammenspiel beider Prinzipien (Rotation und Translation des Körpers 12b) auch kontrollierbare Drehbewegungen (z. B. 360-Grad-Drehungen) des Grundkörpers 10b um die Drehachsen des Gelenks 20b möglich. Anders ausgedrückt kann der Grundkörper 10b ohne Einschränkungen (außer einer gegebenen maximalen und minimalen Krafteinleitungsbedingung in den Gelenken 22b und/oder 20b) frei orientiert werden, wobei sich der Körper 12b beispielsweise nur auf einer linearen Führung bewegt und/oder der Körper 12b zusätzlich rotiert.

Der Körper 12b kann mit dem Ziel, den Grundkörper 10b in eine vorbestimmte neue Ruhelage, welche auch als Sollwert zu verstehen sein kann, zu bringen, verschoben und/oder gedreht werden. Die neue Ruhelage des Grundkörpers 10b wird dabei durch konkrete Werte der Drehwinkel der nicht aktuierten Gelenke 20b und/oder 22b beschrieben. Um die neue Ruhelage zu erreichen, kann eine entsprechend ausgelegte oder konfigurierte Drehratenregelung eingesetzt werden. Bei dieser Drehratenregelung werden jeweils aktuell vorliegende oder eingenommene Drehwinkel und/oder Drehgeschwindigkeiten der Gelenke 20b und/oder 22b gemessen und mit Sollwerten der Drehwinkel, die der Grundkörper 10b in der neuen Ruhelage einnehmen oder aufweisen soll, verglichen bzw. verrechnet. Daraus werden neue Stellgrößen für den aktuierten Körper 12b des Segments 100b abgeleitet.

Die Verschiebung des Körpers 12b kann wie zu Fig. 1 beschrieben erfolgen. Zudem trifft das zu Fig. 1 Beschriebene in einigen erfindungsgemäßen Ausführungsformen auch für die Ausführungen zur Fig. 2 sowie zu den übrigen Figuren zu, wo immer dies für den Fachmann als technisch möglich erkannt wird.

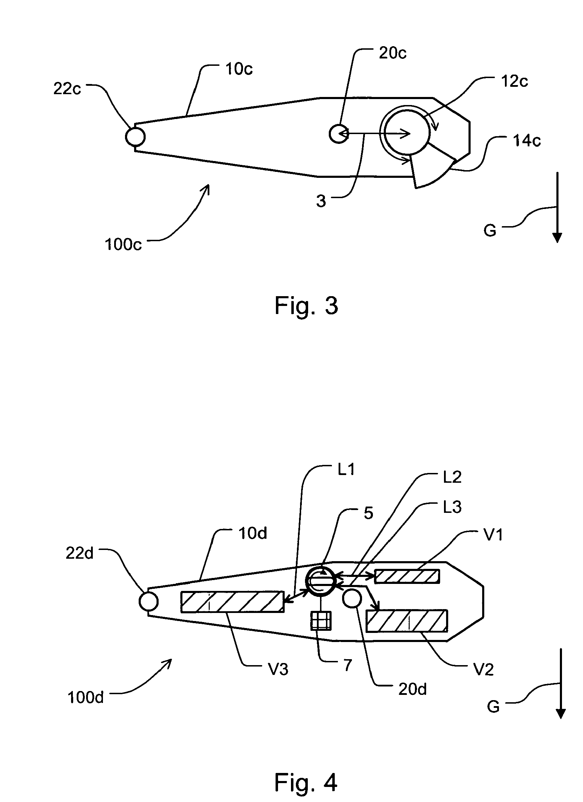

**Fig. 3** zeigt die Darstellung der Fig. 2, zusätzlich jedoch einen weiteren Körper, der als ausgeprägte Unwucht 14c mit dem Körper 12c verbunden ist. Die Unwucht 14c ist z. B. mittels Schraub-, Kleb- oder Schweißverbindung mit dem Körper 12c verbunden.

Durch diese Unwucht 14c verändert sich das Bewegungsverhalten, insbesondere das Drehverhalten und das dynamische Verhalten des Körpers 12c und in deren Folge auch das Gesamtverhalten der Einrichtung 100c.

**Fig. 4** zeigt eine Einrichtung 100d (Segment 100d), mit einem Grundkörper 10d und einer Pumpe 5 mit einem Steuergerät 7 zum Umverteilen von Flüssigkeit zwischen verschiedenen Vorratsvolumina V1, V2, V3 (der Begriff Vorratsvolumina wird synonym zu dem Begriff Speichervolumina verwendet).

Im Unterschied zu den drehbaren und/oder verschiebbaren Körpern 12a, 12b und 12c in den Figuren 1 bis 3 erfolgt in Fig. 4 die Massenverteilung auf hydraulischem Wege. Die Einrichtung 100d weist für diesen Zweck drei Vorratsvolumina V1, V2, V3 (es sind auch weitere Vorratsvolumina möglich, hier werden aus Gründen der vereinfachten Darstellung nur drei gezeigt) auf, die eine Flüssigkeit, z. B. Wasser, Öl oder eine andere Flüssigkeit, beinhalten.

Eine Pumpe 5, die von einem Steuergerät 7 Steuersignale erhält, pumpt mittels entsprechender Leitungen L1 und/oder L2 und/oder L3 Flüssigkeit von wenigstens einem der Vorratsvolumina V1 und/oder V2 und/oder V3 in ein anderes Vorratsvolumen (V1, V2 oder V3). Als Folge dieser Massenverteilung durch Umpumpen von Flüssigkeit kann eine Drehbewegung des Segments 100d einsetzen. Diese Drehbewegung kann so lange andauern, bis sich das Segment 100d wieder im Momentengleichgewicht befindet.

Durch eine geometrisch günstige Anordnung der wenigstens drei Vorratsvolumina V1, V2, V3 ist es im Segment 100d möglich, eine Drehbewegung durch z. B. zyklisches Umpumpen zwischen den einzelnen Vorratsvolumina V1, V2, V3 zu erreichen.

Die Massenumverteilung der Flüssigkeiten in den Vorratsvolumina V1, V2, V3 kann mit dem Ziel, den Grundkörper 10d in eine vorbestimmte neue Ruhelage, auch als Sollwert zu verstehen, zu bringen, erfolgen. Die neue Ruhelage des Grundkörpers 10d wird dabei durch konkrete Werte der Drehwinkel der nicht aktuierten Gelenke 20d und/oder 22d beschrieben. Um die neue Ruhelage zu erreichen, kann eine entsprechend ausgelegte oder konfigurierte Drehratenregelung eingesetzt werden. Bei dieser Drehratenregelung werden die Drehwinkel und/oder Drehgeschwindigkeiten der Gelenke 20d und/oder 22d gemessen und mit Sollwerten der Drehwinkel, die der Grundkörper 10d in der neuen Ruhelage einnehmen oder aufweisen soll, verglichen bzw. verrechnet. Daraus werden neue Stellgrößen für die Umverteilung der Flüssigkeiten in den Vorratsvolumina V1, V2, V3 abgeleitet. Die Stellgrößen können direkt mit der Steuereinheit der Pumpe 7 gekoppelt werden, so dass dadurch ein Regelkreis aufgebaut werden kann.

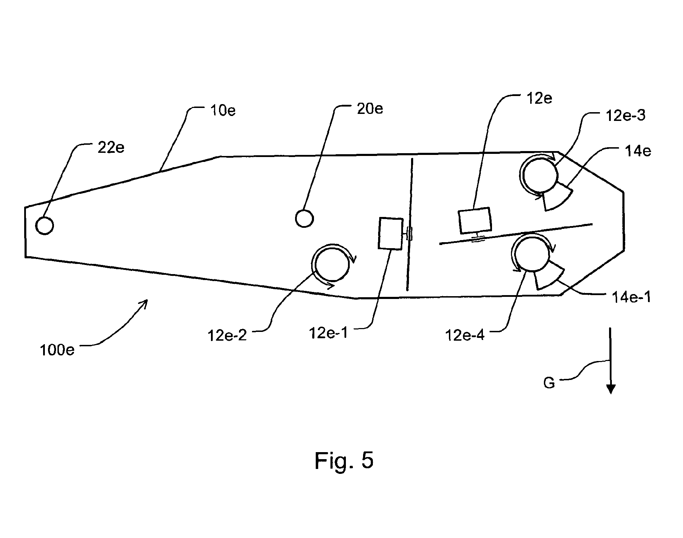

**Fig. 5** zeigt eine Einrichtung 100e (Segment 100e), aufweisend einen Grundkörper 10e und verschiedene Abschnitte (Abschnitte werden im Folgenden als Körper bezeichnet), wie sie in den Figuren 1 bis 3 dargestellt wurden.

Die verschiedenen Körper weisen in diesem Ausführungsbeispiel translatorisch gelagerte Körper 12e und 12e-1 auf, einen drehbaren Körper 12e-2 sowie zwei weitere drehbare Körper 12e-3 und 12e-4 mit jeweils einer ausgeprägten Unwucht (14e, 14e-1). Diese Körper sind hier rein exemplarisch dargestellt, die Einrichtung 100e kann erfindungsgemäß weitere Körper einschließen.

Die verschiedenen Körper werden in der Einrichtung 100e parallel eingesetzt, das heißt die Körper können alle oder teilweise gleichzeitig bewegt werden.

Beispielsweise können die linear aktuierten Körper (12e und/oder 12e-1) bewegt werden und dadurch Drehungen der Einrichtung 100e bewirken.

Im Folgenden wird eine Bewegungsabfolge beschrieben, bei der mittels des Körpers 12e eine 360-Grad-Drehung des Grundkörpers 10e erfolgt. Das System ist wie in Fig. 5 dargestellt zunächst in Ruhe. Der Körper 12e bewegt sich anschließend vom Gelenk 20e weg (in Fig. 5 nach rechts), woraufhin der Grundkörper 10e beginnt, sich im Uhrzeigersinn um das Gelenk 20e zu drehen und sich bei einer Drehung von ca. 90 Grad aufzurichten. Diese Drehung wird über die 270 Grad hinaus weiter ausgeführt. Während dieser über 270 Grad hinaus weiter ausgeführten Drehung wird der Körper 12e wieder in die ursprüngliche Lage (wie in Fig. 5 gezeigt) zurückbewegt (aktuiert). Dabei kann die ursprüngliche Lage des Grundkörpers 10e (wie in Fig. 5 gezeigt) zunächst zum Abbremsen durchlaufen werden, bevor der Grundkörper 10e (der Körper 12e ist bereits wieder in der Ausgangslage) wieder zurückschwingt und sich erst dann in die Ausgangslage zurückbewegt.

Diese Ausgangsruhelage wird auch dann wieder nach einer 360-Grad-Drehung erreicht, wenn der Schwerpunkt des Körpers 12e einerseits und der Gesamtschwerpunkt der restlichen Körper andererseits nicht mit der Drehachse 20e des Segments 100e auf einer Ebene liegen (sogenannte Wägekinematik). Je nach vorhandenen Reibwerten zwischen den bewegten Körpern (Lagerreibung etc.) kann auch eine Ruhelage erreicht werden, die von der Ausgangsruhelage abweicht. Weiterhin kann das Erreichen einer Ruhelage durch ein aperiodisches Einschwingen mithilfe entsprechender Reglerparametrisierung unterstützt werden.

Da die Körper 12e und 12e-1 bezüglich ihrer Translationsrichtung unterschiedlich orientiert sind, kann die Situation eines sogenannten (unteren) Totpunkts des Segments 100e nicht auftreten. Dies gilt insbesondere für Fälle, in denen eine entsprechende Ansteuerung vorgesehen ist.

Bei einem (unteren) Totpunkt befindet sich der Körper 12e genau unterhalb des Achsmittelpunkts des Gelenks 20e. Bei diesem sogenannten "Toten Punkt" wird trotz Aktuierung des Körpers 12e kein Moment auf den Grundkörper 10e übertragen, so dass keine Rotation des Segments 100e ausgeführt wird.

Die Körper 12e, 12e-1, 12e-2, 12e-3, 12e-4 können mit dem Ziel, den Grundkörper 10e in eine vorbestimmte neue Ruhelage, auch als Sollwert zu verstehen, zu bringen, verschoben und/oder gedreht werden. Die neue Ruhelage des Grundkörpers 10e wird dabei durch konkrete Werte der Drehwinkel der nicht aktuierten Gelenke 20e und/oder 22e beschrieben. Um die neue Ruhelage zu erreichen, kann eine entsprechend ausgelegte oder konfigurierte Drehratenregelung eingesetzt werden. Bei dieser Drehratenregelung werden die Drehwinkel und/oder Drehgeschwindigkeiten der Gelenke 20e und/oder 22e gemessen und mit Sollwerten der Drehwinkel, die der Grundkörper 10e in der neuen Ruhelage einnehmen oder aufweisen soll, verglichen bzw. verrechnet. Daraus werden neue Stellgrößen für die aktuierten Körper 12e, 12e-1, 12e-2, 12e-3, 12e-4 des Segments 100e abgeleitet.

**Fig. 6** zeigt eine kinematische Kette 1000, welche drei Einrichtungen (Segmente) 100a, 100a-1, 100a-2 entsprechend Fig. 1 aufweist.

Die kinematische Kette 1000 ist an einem Untergrund 200 (z. B. Boden, Platte etc.) fixiert mit einem (rotatorischen) Freiheitsgrad.

Den Anfang der kinematischen Kette 1000 bildet das Segment 100a-1 mit dem Gelenk 20a-1 (hier als nicht aktuiertes Drehgelenk des Segments 100a-2 dargestellt) und dem aktuierten Körper 12a-1.

Den zweiten Teil der kinematischen Kette 1000 bildet das Segment 100a mit den Gelenken 20a und 22a sowie dem Grundkörper 10a und dem translatorisch verschiebbaren, aktuierten Körper 12a. Das Gelenk 20a ist in diesem Fall deckungsgleich mit dem Gelenk 22a-1 des Segments 100a-1.

Den dritten und letzten Teil der kinematischen Kette 1000 bildet das Segment 100a-2 mit dem nicht aktuierten Gelenk 22a.

Bei Ruhelage der kinematische Kette 1000 stellt sich der Neigungswinkel 9 des Segments 100a gegenüber der Horizontalen ein.

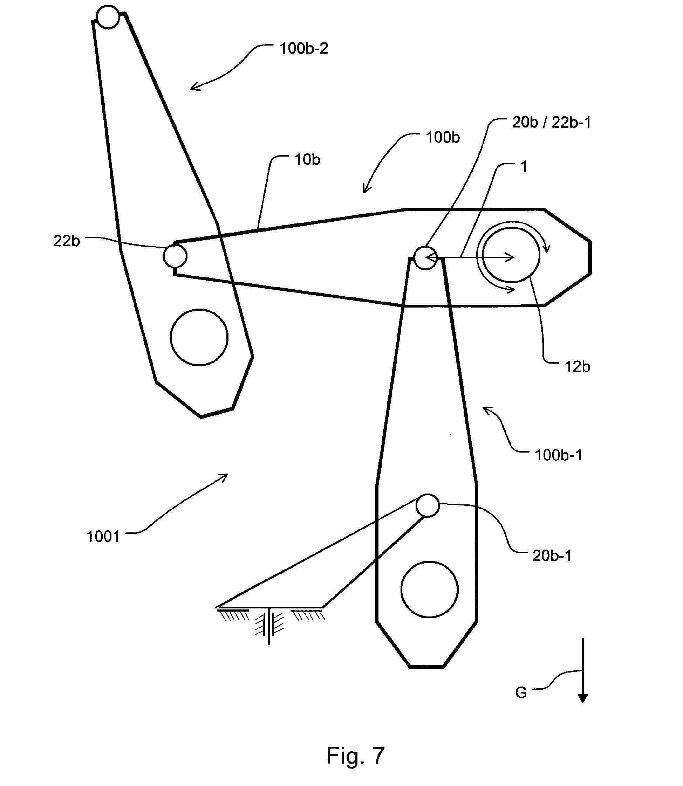

**Fig. 7** zeigt eine weitere kinematische Kette 1001, aufweisend drei Einrichtungen (Segmente) 100b, 100b-1, 100b-2 entsprechend Fig. 2.

Das zu den oben diskutierten Figuren und insbesondere zu Fig. 2 Beschriebene trifft in einigen erfindungsgemäßen Ausführungsformen auch auf die Ausführungen zur Fig. 7 zu, wo immer dies für den Fachmann als technisch möglich erkannt wird.

Den Anfang der kinematischen Kette 1001 bildet das Segment 100b-1 mit dem Gelenk 20b-1 (hier als nicht aktuiertes Drehgelenk des Segments 100b-1).

Den zweiten Teil der kinematischen Kette 1001 bildet das Segment 100b mit den Gelenken 20b und 22b sowie dem Grundkörper 10b und dem aktuierten Körper 12b, der als rotatorisch und translatorisch gelagerter Körper ausgestaltet ist.

Den dritten und letzten Teil der kinematischen Kette 1001 bildet das Segment 100b-2.

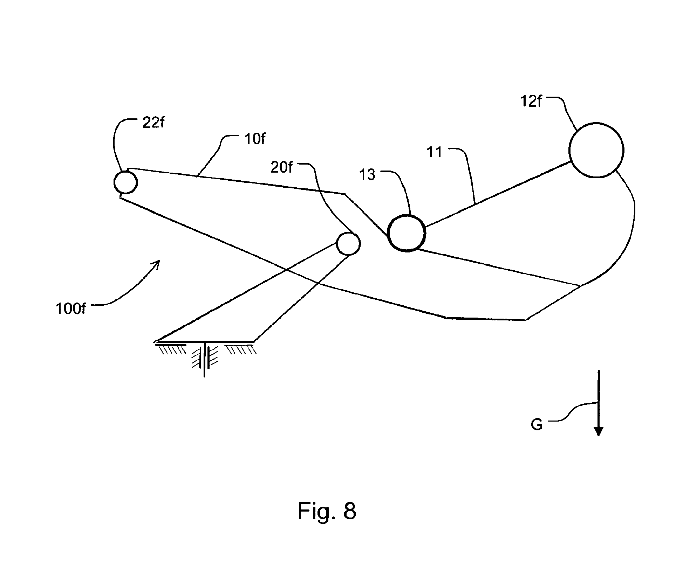

**Fig. 8** zeigt eine elastisch deformierbare Einrichtung 100f (Segment 100f) mit einem Grundkörper 10f und einem Abschnitt 12f (Körper 12f).

Der Körper 12f ist hier als Rundkörper (z. B. Kugel) dargestellt. Der Körper 12b kann jedoch auch als sogenannte Massekonzentration stellvertretend für weitere und/oder anders ausgestaltete, hier nicht gezeigte Körper betrachtet werden.

Der Körper 12f kann auf verschiedene Weisen mit dem Grundkörper 10f verbunden sein. Er kann beispielsweise direkt mit dem zu einem Blech auslaufenden Grundkörper 10f verschweißt oder anderweitig verbunden sein. Der Körper 12f kann mechanisch wieder lösbar (demontierbar), z. B. mittels einer Schraubverbindung, mit dem Grundkörper 10f verbunden sein.

Eine weitere Möglichkeit einer Verbindung zwischen dem Körper 12f und dem Grundkörper 10f ist eine mechanische Kopplung. Diese kann beispielsweise mittels eines Seilzugs 11 oder einer Kette oder dergleichen mit einem Aktor 13, der den Seilzug 11 betätigt (z. B. auf einer Rolle aufwickelt), verwirklicht sein. Ebenfalls möglich ist eine Kopplung mittels eines Hydraulikzylinders (hier nicht dargestellt) sowie eines antreibenden Aktors 13 (dies kann ein Motor, Stellmotor, Schrittmotor etc. sein).

Mittels einer dieser mechanischen Verbindungsvarianten kann die Lage des Grundkörpers 10f relativ zum Körper 12f verändert werden. Hierdurch verändert sich die Lage des resultierenden Schwerpunktes des Segments 100f, und infolgedessen tritt eine Drehbewegung in Richtung einer neuen Ruhelage gemäß den Gesetzen der Mechanik (Momentengleichgewicht etc.) ein. Wird bei einer Verformung (des Grundkörpers 10f und/oder eines verbindenden Elements mit dem Körper 12f) nicht die Elastizitätsgrenze des verwendeten Materials überschritten, so ist der Vorgang reversibel und der Körper 12f kann wieder in die Ausgangslage zurückbewegt werden.

Der Körper 12f kann mit dem Ziel, den Grundkörper 10f in eine vorbestimmte neue Ruhelage, auch als Sollwert zu verstehen, zu bringen, in seiner räumlichen Position verändert werden. Letzteres kann beispielsweise durch elastische Verformungen eines verbindenden Elements zwischen dem Grundkörper 10f und dem Körper 12f erfolgen). Die neue Ruhelage des Grundkörpers 10f wird dabei durch konkrete Werte der Drehwinkel der nicht aktuierten Gelenke 20f und/oder 22f beschrieben. Um die neue Ruhelage zu erreichen, kann eine entsprechend ausgelegte oder konfigurierte Drehratenregelung eingesetzt werden. Bei dieser Drehratenregelung werden die Drehwinkel und/oder Drehgeschwindigkeiten der Gelenke 20f und/oder 22f gemessen und mit Sollwerten der Drehwinkel, die der Grundkörper 10f in der neuen Ruhelage einnehmen oder aufweisen soll, verglichen bzw. verrechnet. Daraus werden neue Stellgrößen für den aktuierten Körper 12f des Segments 100f abgeleitet.

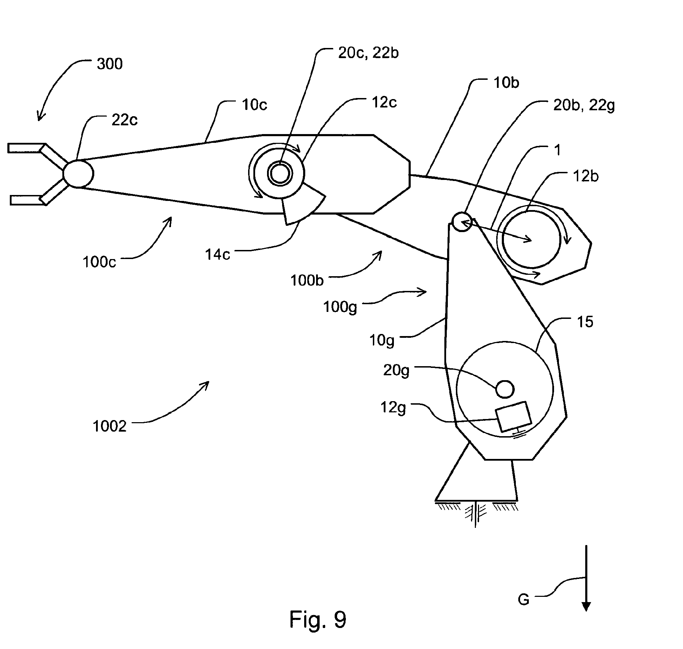

**Fig. 9** zeigt eine weitere kinematische Kette 1002. Diese hat drei Einrichtungen (100b, 100c, 100g) bzw. Segmente und einen Manipulator 300 (synonym zu Greifarm). Letzterer ist am Gelenk 22c des Segments 100c fixiert.

Das Segment 100b wurde bereits in Fig. 2 und das Segment 100c in Fig. 3 dargestellt und beschrieben (einschließlich der konstruktiv gegebenen aktuierten Möglichkeit des Umkonfigurierens der Massenverteilungen), worauf hiermit Bezug genommen wird.

Die kinematische Kette 1002 mit den drei Segmenten (100b, 100c, 100g) stellt eine serielle Kinematik dar, wobei das Segment 100c am Ende und das Segment 100g am Anfang oder inertialen Anfang der kinematischen Kette 1002 angeordnet ist.

Das Segment 100c umfasst den Grundkörper 10c und den um das Gelenk 20c gelagerten und relativ zum Grundkörper 10c aktuierten Körper 12c. Der Körper 12c weist eine ausgeprägte Unwucht 14c auf.

Das Segment 100c kann derart ausgelegt werden oder sein, dass der Schwerpunkt des Grundkörpers 10c in der Drehachse 20c unter Berücksichtigung der in 22c anfallenden Kräfte und ggf. Momente liegt. In diesem Fall bedeutet dies eine für alle räumlichen Positionen des Segments 100c stabile Lage des Grundkörpers 10c. Dies setzt voraus, dass auf den Körper 12c kein Moment wirkt (d. h., dass der Körper nicht aktuiert ist) und als angreifende Kraft die Schwerkraft des Schwerefeldes G wirkt.

Soll die räumliche Position des Grundkörpers 10c verändert werden, so kann ein zwischen dem Grundkörper 10c und dem Körper 12c sitzender Aktor (hier nicht dargestellt) ein Moment aufbringen, so dass der Grundkörper 10c und der Körper 12c gemäß ihrer Trägheitsmomentenverhältnisse gegeneinander verdreht werden. Da dabei der Schwerpunkt des Körpers 12c aus der lotrechten Ebene durch die Achse des Gelenks 20c gerät, beginnt sich der Grundkörper 10c so lange in eine Richtung zu drehen oder zu beschleunigen, bis der Schwerpunkt des Körpers 12c wieder unter der lotrechten Ebene steht.

Das Segment 100b umfasst den Grundkörper 10b und den rotatorisch und translatorisch gelagerten Körper 12b. Das Segment 100b kann auf zwei Arten eine kontrollierte Bewegung, hier eine Rotation um die Achse des Gelenks 20b, ausführen. Zum einen aufgrund von einer translatorischen Bewegung des Körpers 12b und zum anderen durch eine rotatorische Bewegung und Beschleunigung des Körpers 12b. Beide Möglichkeiten werden im Folgenden dargestellt.

Für beide Möglichkeiten können die bewegten Körper (Grundkörper 10b und Körper 12b) bezüglich ihrer Massenverhältnisse derart ausgelegt werden oder sein, dass der gemeinsame Schwerpunkt des Grundkörpers 10b und des Körpers 12b unter Berücksichtigung des Segments 100c mit Anbauteilen (hier dem Manipulator 300) und bei einer Auslenkung des Körpers 12b um einen vorgegebenen Weg bzw. Winkel in die Achse des Gelenks 20b fällt.

Die erste Möglichkeit zur Rotation des Segments 100b besteht darin, den Körper 12b translatorisch auf dem Verschiebeweg 1 (Abstand zwischen der Drehachse des Körpers 12b und dem Gelenk 20b) zu bewegen. Dadurch wird das Momentengleichgewicht um das Gelenk 20b gestört und das Segment 100b wird rotatorisch bewegt und beschleunigt.

Wird der Körper 12b anschließend wieder in die Ausgangslage bewegt, bleibt die Rotationsgeschwindigkeit bei idealen Bedingungen (keine Reibung etc.) erhalten. Allerdings kann bei der hier dargestellten Geometrie, insbesondere der translatorischen Verschieberichtung (Verschiebweg 1), der Fall auftreten, dass sich der Körper 12b genau unterhalb (in Richtung des Schwerefeldes G) des Achsmittelpunkts des Gelenks 20b befindet. Bei diesem sogenannten "Toten Punkt" wird trotz Aktuierung des Körpers 12b kein Moment auf den Grundkörper 10b übertragen, so dass keine Rotation des Segments 100b ausgeführt wird.

Aufgrund der Problematik des "Toten Punkts" ist es auch möglich, die zweite Möglichkeit zur Rotation des Segments 100b zu nutzen. Dabei wird die Translation des Körpers 12b entlang des Verschiebewegs 1 nur zur Austarierung des Gleichgewichts verwendet, während die rotatorische Beschleunigung des Körpers 12b dazu genutzt wird, um in den Grundkörper 10b ein Moment zur Drehbeschleunigung einzuleiten.

Das Segment 100g weist den Grundkörper 10g und den Körper 12g auf. Der Körper 12g, der hier als translatorisch gelagerter Körper dargestellt ist, wird auf einer geschlossenen Kurvenbahn 15 geführt. Die Achse des Gelenks 20g wird von der Kurvenbahn 15 eingeschlossen. Durch die Führung durch die Kurvenbahn 15 und eine gezielte Dimensionierung der Massenverhältnisse des Körpers 12g und des Grundkörpers 10g kann sich das Segment 100g frei drehen (z. B. um 360 Grad), ohne einen Kraftschluss mit den Teilen zu benötigen, die sich außerhalb des Segments 100g befinden.

Jeder der Körper 12f, 12g kann mit dem Ziel, den jeweiligen Grundkörper 10f, 10g in eine vorbestimmte neue Ruhelage, auch als Sollwert zu verstehen, zu bringen, verschoben und/oder gedreht werden. Die jeweils neue Ruhelage des Grundkörpers 10f, 10g wird dabei durch konkrete Werte der Drehwinkel der nicht aktuierten Gelenke 20b und/oder 20g und/oder 22g beschrieben. Um die neue Ruhelage zu erreichen, kann eine entsprechend ausgelegte oder konfigurierte Drehratenregelung eingesetzt werden. Bei dieser Drehratenregelung werden die Drehwinkel und/oder Drehgeschwindigkeiten der Gelenke 20b und/oder 20g und/oder 22g gemessen und mit Sollwerten der Drehwinkel, die der jeweilige Grundkörper 10f, 10g in der neuen Ruhelage einnehmen oder aufweisen soll, verglichen bzw. verrechnet. Daraus werden neue Stellgrößen für den jeweils aktuierten Körper 12b und/oder 12g des jeweiligen Segments 100b bzw. 100g abgeleitet.

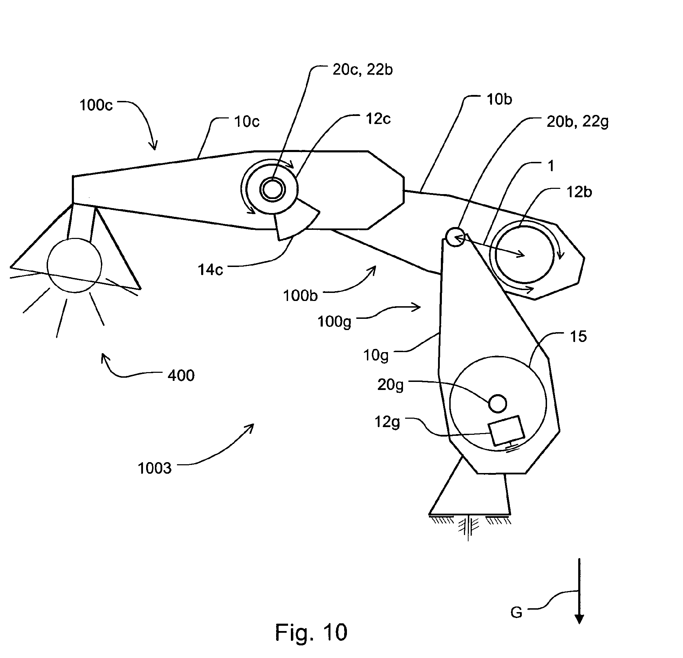

**Fig. 10** zeigt eine weitere kinematische Kette 1003, aufweisend drei Einrichtungen 100c, 100b, 100g mit einer Leuchte 400.

Die kinematische Kette 1003 ist in der gleichen Weise aufgebaut wie die kinematische Kette 1002 aus Fig. 9. Daher wird auf die Beschreibung zu Fig. 9 verwiesen.

Die Leuchte 400 kann beispielsweise eine Tischlampe sein, wobei die kinematische Kette 1003 an einem Tisch (Schreibtisch, Arbeitstisch, Feinmontagetisch etc.) fixiert werden kann.

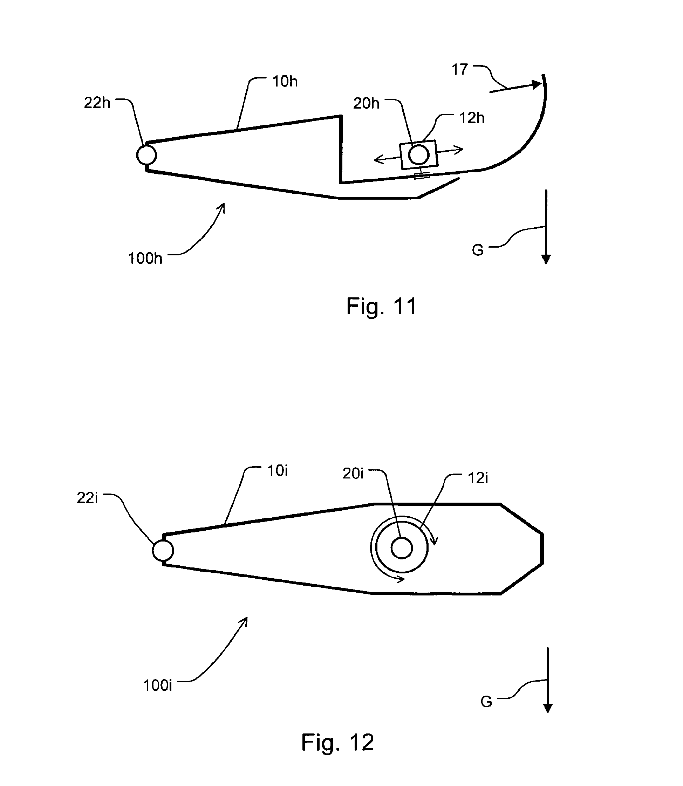

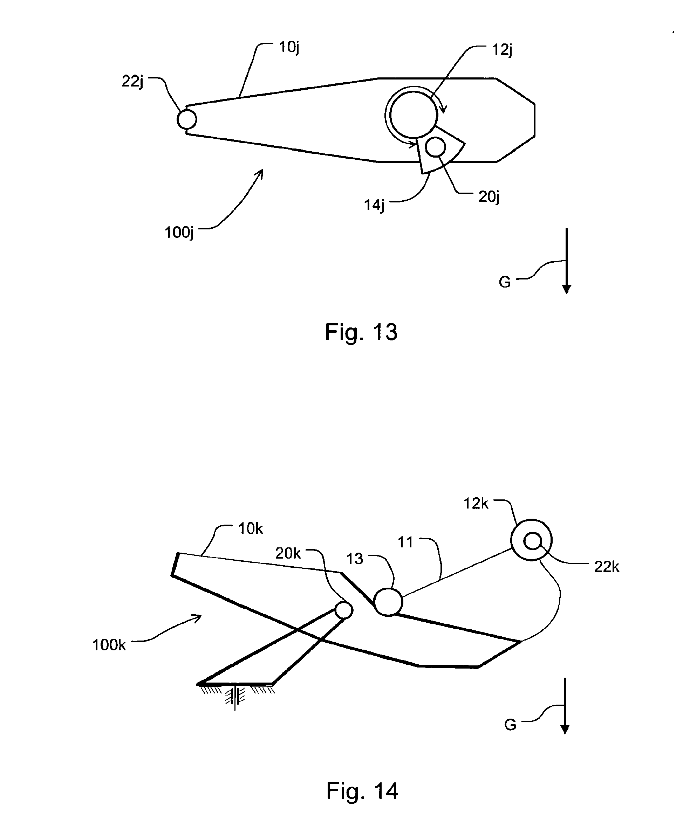

**Fig. 11** bis **Fig. 14** zeigen die Fig. 1, Fig. 2, Fig. 3 und Fig. 8, jedoch mit Segmenten 100h, 100i, 100j und 100k, die variable Achsabstände zwischen den beiden Gelenken 20h, 20i, 20j, 20k einerseits und 22h, 22i, 22j, 22k andererseits aufweisen.

In den Fig. 1, Fig. 2, Fig. 3 und Fig. 8 sind die Achsabstände konstant, da die Gelenke jeweils auf den Grundkörpern (10a, 10b, 10c, 10k) fixiert sind. In den Fig. 11 bis Fig. 14 dagegen sind lediglich die Achsen der Gelenke 22h, 22i, 22j und 22k auf den Grundkörpern 10h, 10i, 10j und 10k fixiert, die Achsen der Gelenke 20h, 20i, 20j und 20k dagegen jedoch auf den beweglichen Körpern 12h, 12i, 12j und 12k. Die Achsen der Gelenke 20h, 20i, 20j, 20k, 22h, 22i, 22j und 22k sind nicht aktuiert. Die Wirkweisen der Segmente 100h, 100i, 100j und 100k entsprechen denen in Fig. 1, Fig. 2, Fig. 3 und Fig. 8.

Die Körper 12h, 12i, 12j und 12k können mit dem Ziel, den jeweiligen Grundkörper 10h, 10i, 10j, 10k in eine vorbestimmte neue Ruhelage, auch als Sollwert zu verstehen, zu bringen, verschoben und/oder gedreht und/oder bewegt werden. Die neue Ruhelage des jeweiligen Grundkörpers 10h, 10i, 10j, 10k wird dabei durch konkrete Werte der Drehwinkel der nicht aktuierten Gelenke 20h, 20i, 20j, 20k und/oder 22h, 22i, 22j und 22k beschrieben. Um die neue Ruhelage zu erreichen, kann eine entsprechend ausgelegte oder konfigurierte Drehratenregelung eingesetzt werden. Bei dieser Drehratenregelung werden die Drehwinkel und/oder Drehgeschwindigkeiten der Gelenke 20h, 20i, 20j, 20k und/oder 22h, 22i, 22j und 22k gemessen und mit Sollwerten der Drehwinkel, die der jeweilige Grundkörper 10h, 10i, 10j, 10k in der neuen Ruhelage einnehmen oder aufweisen soll, verglichen bzw. verrechnet. Daraus werden neue Stellgrößen für den aktuierten Körper 12h, 12i, 12j und 12k des jeweiligen Segments 100h, 100i, 100j und 100k abgeleitet.

**Fig. 15** zeigt eine Anwendung der kinematischen Kette 1003 aus Fig. 10 zur Positionierung von zwei Operationsleuchten 400 (abgekürzt OP-Leuchten 400) in einem Operationsraum während einer Operation. Die zwei dargestellten kinematischen Ketten 1003 sind an der Decke 25 des Operationsraumes befestigt.

Ein Chirurg 500 operiert einen Patienten 600, der auf einem Operationstisch 700 gelagert ist. Der Chirurg 500 operiert mit einem Skalpell 19.

In diesem Ausführungsbeispiel wird die Position und die Führung des Skalpells 19 durch einen Infrarotstrahl 21 (abgekürzt IR-Strahl) nachverfolgt. Der IR-Strahl 21 wird von einem Sender 23 am Kopf des Chirurgen ausgesendet bzw. emittiert.

Der IR-Strahl 21 und damit die Blickrichtung des Chirurgen 500 wird von einem Kamerasystem 800 erfasst, das in diesem Beispiel an der Decke 25 des Operationsraumes befestigt ist. Die mithilfe des Kamerasystems 800 erfassten Signale des IR-Strahls 21 können als Eingangssignale für eine Regelung oder Steuerung der Körper 12 der Segmente 100 (siehe auch die vorangegangenen Figuren) der kinematischen Ketten 1003 genutzt werden. Damit kann eine Nachführung der OP-Leuchten 400 auf das jeweilige Operationsfeld des Chirurgen 500 erreicht werden, auf das der Chirurg 500 jeweils sieht.

Mit derartigen OP-Leuchten 400 kann eine 3-dimensionale Ausleuchtung des Operationsfeldes erreicht werden. Die Regelung, die hier beispielhaft durch den IR-Strahl 21 und das Kamerasystem 800 dargestellt ist, kann dynamisch nachgeregelt werden.

Anstelle der hier dargestellten Regelung, welche ein an der Decke 25 befestigtes Kamerasystem 800 aufweist oder nutzt, kann die Regelung (oder Steuerung, dann ohne geschlossenen Regelkreis) der kinematischen Kette 1003 alternativ mittels eines sogenannten "Eye-Tracking-Systems" (System zur Blickerfassung) erfolgen. Bei diesem System wird die Blickrichtung des Chirurgen 500 mittels geeigneter Sensoren, z. B. optischer Sensoren am Kopf des Chirurgen 500, ermittelt. Diese Informationen werden weiterverarbeitet und als Eingangssignale für die Körper 12 der Segmente 100 der kinematischen Ketten 1003 genutzt. "Eye-Tracking-Systeme" sind dem Fachmann bekannt.

Alternativ zu der Regelung mit dem Kamerasystem 800 oder mit dem "Eye-Tracking-System" kann eine Inertial-Messeinheit verwendet werden. Bei dieser werden translatorische und/oder rotatorische Beschleunigungskräfte am Kopf des Chirurgen 500 gemessen. Diese sind für oder als Eingangssignale zur Regelung oder Steuerung der kinematischen Kette 1003 nutzbar. Diese Ausführung ist in Fig. 15 nicht dargestellt.

Anstelle des Kamerasystems 800 an der Decke 25 des Operationsraumes kann eine Kamera (oder ein sogenannter IR-Suchkopf) direkt am oder in unmittelbarer Nähe des IR-Strahl-Senders 23 installiert werden. Dieser IR-Suchkopf weist in der Regel drei IR-Empfänger (Detektoren) auf. Die hierüber empfangenen Signale können als Eingangssignale zur Regelung oder Steuerung der kinematischen Ketten 1003 genutzt werden. Diese Ausführung ist in Fig. 15 nicht dargestellt.

**Fig. 16** zeigt eine weitere Anwendung der kinematischen Kette 1003 aus Fig. 10 zur Positionierung einer Operationsleuchte 400 und eines Greifarms 300.

Dieses Ausführungsbeispiel entspricht weitgehend der Fig. 15 und deren Beschreibung, weist jedoch anstelle der zweiten OP-Leuchte 400 einen Manipulator 300 (oder Greifarm 300) auf. Mit diesem Greifarm 300 kann beispielsweise eine Zugentlastung von Schläuchen, Kabeln oder ähnlichem durchgeführt werden. Derartige Schläuche oder Kabel können ein Gerät, das der Chirurg 500 bei der Operation verwendet, mit einer ortsfesten Basisstation, einem Netzteil, oder ähnlichem verbinden.

Weitere Einsatzmöglichkeiten für den Greifarm 300 können Operationshilfen sein, wie beispielsweise das Halten von Gefäßen oder Organen, an denen der Chirurg 500 operiert, oder welche aus dem Operationsgebiet heraus gehalten werden sollen.

## Patentansprüche

1. Einrichtung (100) mit wenigstens einem Gelenk (20, 22), welche um das Gelenk (20, 22) drehbar in einem Schwerefeld (G) gelagert ist, wobei
- die Einrichtung (100) wenigstens einen ersten Abschnitt (10) aufweist; und
- die Einrichtung (100) wenigstens einen zweiten Abschnitt (12) aufweist, der relativ zum ersten Abschnitt (10) bewegbar ist;
**dadurch gekennzeichnet, dass**
- der zweite Abschnitt (12) zum Generieren einer Drehbewegung der Einrichtung (100) um das Gelenk (20, 22) ausgestaltet ist.

2. Einrichtung (100) nach Anspruch 1, wobei die Einrichtung (100) Mittel zur Erzeugung von kontrollierbaren oder kontrollierten Beschleunigungen der Einrichtung (100) aufweist.

3. Einrichtung (100) nach Anspruch 1 oder 2, mit einem Mittel zum aktiven Verändern, Steuern oder Regeln der Lage des bewegbaren zweiten Abschnitts (12) relativ zum ersten Abschnitt (10).

4. Einrichtung (100) nach einem der vorangegangenen Ansprüche, wobei der bewegbare zweite Abschnitt (12) relativ zum ersten Abschnitt (10) verschiebbar und/oder rotierbar ist.

5. Einrichtung (100) nach einem der vorangegangenen Ansprüche, welche wenigstens zwei bewegbare zweite Abschnitte (12) aufweist, welche miteinander verbunden sind.

6. Einrichtung (100) nach einem der vorangegangenen Ansprüche, wobei die Einrichtung (100) wenigstens ein aktuiertes Gelenk (20, 22) aufweist.

7. Verfahren zum Bewegen einer Einrichtung (100), insbesondere nach einem der Ansprüche 1 bis 6, welche wenigstens ein Gelenk (20, 22) aufweist, und welche um das Gelenk (20, 22) drehbar in einem Schwerefeld (G) gelagert ist, wobei
- die Einrichtung (100) wenigstens einen ersten Abschnitt (10) aufweist; und
- die Einrichtung (100) wenigstens einen zweiten Abschnitt (12) aufweist, der relativ zum ersten Abschnitt (10) bewegbar ist;
wobei das Verfahren den Schritt umfasst:
- Verändern der Lage des zweiten Abschnitts (12) relativ zum ersten Abschnitt (10) zum Generieren einer Drehbewegung der Einrichtung (100) um das Gelenk (20, 22).

8. Verfahren nach Anspruch 7, mit den Schritten:
- Bestimmen von Winkeln und/oder Positionen wenigstens eines der Gelenke (20, 22) der Einrichtung (100);
- Vergleichen dieser Winkel und/oder Positionen mit vorgegebenen Sollwerten;
- Bestimmen einer Abweichung zwischen den zuvor bestimmten Werten und den Sollwerten; und
- Verändern der Positionen und/oder Geschwindigkeiten des ersten und des zweiten Abschnitts (10, 12) relativ zueinander unter Berücksichtigung der Abweichung.

9. Verfahren nach einem der Ansprüche 7 oder 8, mit den Schritten:
- Bestimmen der Winkel und/oder Winkelgeschwindigkeiten wenigstens eines der nicht aktuierten Gelenke (20, 22) der Einrichtung (100);
- Bestimmen einer Differenz zwischen einem vorgegebenen Sollwert einer Aktorstellgröße und einer aktuellen Aktorstellgröße;
- Verstärken dieser Differenz in proportionaler und/oder integraler und/oder differentieller Form;
- Erzeugen von Steuersignalen für die aktuierten Abschnitte der Einrichtung (100) aufgrund der bestimmten Winkel und/oder Winkelgeschwindigkeiten und/oder bestimmten Differenzen; und
- Ansteuern der aktuierten Abschnitte (12) der Einrichtung (100) mit den erzeugten Steuersignalen.

10. Steuereinrichtung, geeignet und vorgesehen zum Ausführen eines Verfahrens nach einem der Ansprüche 7 bis 9.

11. Vorrichtung (1000), umfassend wenigstens eine Einrichtung (100) nach einem der Ansprüche 1 bis 6, konfiguriert zum Ansteuern von nicht aktuierten Gelenken (20, 22) in der Einrichtung (100) nach einem der Ansprüche 7 bis 9.

12. Medizinische Leuchte (400), vorgesehen zur Anwendung in einem Operationsraum, umfassend wenigstens eine Einrichtung (100) nach einem der Ansprüche 1 bis 6, und/oder umfassend eine Steuereinrichtung gemäß Anspruch 10 und/oder umfassend wenigstens eine Vorrichtung (1000) nach Anspruch 11.

13. Digitales Speichermedium, insbesondere Diskette, CD oder DVD, mit elektrisch auslesbaren Steuersignalen, die derart mit einem programmierbaren Computersystem zusammenwirken können, dass die Schritte eines Verfahrens gemäß einem der Ansprüche 7 bis 9 veranlasst werden.

14. Computerprogramm-Produkt mit auf einem maschinenlesbaren Träger gespeichertem Programmcode zur Veranlassung der Schritte des Verfahrens gemäß einem der Ansprüche 7 bis 9, wenn das Computerprogramm-Produkt auf einem Rechner abläuft.

15. Computerprogramm mit Programmcode zur Veranlassung der Schritte des Verfahrens gemäß einem der Ansprüche 7 bis 9, wenn das Computerprogramm auf einem Computer abläuft.
